Europäisches Patentamt

European Patent Office

Office européen des brevets

(19)

(11) **EP 0 861 821 B1**

(12) **FASCICULE DE BREVET EUROPEEN**

(45) Date de publication et mention
de la délivrance du brevet:
**20.09.2000 Bulletin 2000/38**

(51) Int Cl.7: **C07C 57/04**, C07C 51/16

(21) Numéro de dépôt: **98400314.5**

(22) Date de dépôt: **11.02.1998**

(54) **Procédé de fabrication d'acide acrylique à partir de l'acroléine par réaction redox**

Verfahren zur Herstellung von Acrylsäuren aus Acrolein durch Redox-Reaktion

Process for the preparation of acrylic acid from acroleine by redox reaction

(84) Etats contractants désignés:
**AT BE CH DE DK ES FI FR GB GR IE IT LI LU NL PT SE**

(30) Priorité: **27.02.1997 FR 9702344**

(43) Date de publication de la demande:
**02.09.1998 Bulletin 1998/36**

(73) Titulaire: **Atofina
92800 Puteaux (FR)**

(72) Inventeurs:
• **Hecquet, Gérard
62400 Bethune (FR)**
• **Schirmann, Jean-Pierre
75011 Paris (FR)**
• **Simon, Michel
57500 Saint-Avold (FR)**
• **Pham, Charlotte
67700 Saverne (FR)**

(74) Mandataire: **Rieux, Michel et al
Atofina
D.C.R.D./D.P.I.
4, Cours Michelet,
La Défense 10
92091 Paris la Défense Cedex (FR)**

(56) Documents cités:
EP-A- 0 756 894        EP-A- 0 758 562
DE-A- 4 335 973        FR-A- 2 222 349

EP 0 861 821 B1

**Description**

La présente invention concerne la fabrication de l'acide acrylique à partir de l'acroléine par oxydation selon une réaction redox. L'invention concerne également l'utilisation d'une composition solide d'oxydes mixtes comme système redox pour ladite réaction.

La production industrielle de l'acide acrylique est à l'heure actuelle effectuée par oxydation catalytique de l'acroléine en phase vapeur. Tous les efforts de perfectionnement de ce procédé ont porté jusqu'ici sur la mise au point de catalyseurs donnant un taux de conversion le plus élevé possible de l'acroléine ainsi qu'une sélectivité la plus élevée possible pour l'acide acrylique désiré.

Ainsi, le brevet français n° 2 222 349 décrit un catalyseur pour la préparation de l'acide acrylique par oxydation catalytique en phase vapeur d'acroléine par un gaz contenant de l'oxygène moléculaire, ce catalyseur comprenant un oxyde catalytique sur un support poreux inerte, cet oxyde catalytique ayant la composition en métaux suivante :

$$Mo_{12}V_{2-14}Z_{0,1-6}W_{0-12}Cu_{0-6}$$

Z étant au moins l'un parmi Be, Mg, Ca, Ba et Sr et au moins l'un parmi W et Cu étant toujours présent.

On peut préparer ce catalyseur en ajoutant un support (produit pulvérisé ou billes d'$\alpha$-alumine, de carbure de silicium, etc.) à une solution aqueuse dans laquelle sont dissous des composés des différents éléments du catalyseur, en faisant évaporer la solution aqueuse jusqu'à siccité pour faire déposer les éléments du catalyseur sur le support, et en calcinant le produit séché entre 300 et 800°C.

La Société déposante a maintenant découvert que l'on peut fabriquer l'acide acrylique par oxydation de l'acroléine en phase gazeuse en l'absence d'oxygène moléculaire, en faisant passer un mélange gazeux d'acroléine et de vapeur d'eau et, le cas échéant, d'un gaz inerte sur une composition solide d'oxydes mixtes particulière, laquelle agit comme système redox et fournit l'oxygène nécessaire à la réaction.

Les avantages de ce nouveau procédé sont les suivants :

- l'inconvénient d'une oxydation avec l'oxygène moléculaire est la suroxydation favorisant la dégradation des produits formés ; selon la présente invention, du fait que l'on opère en l'absence d'oxygène moléculaire, la formation de $CO_x$ (monoxyde de carbone et dioxyde de carbone), produits de dégradation, est réduite, ce qui permet d'augmenter nettement la sélectivité en acide acrylique ;
- la sélectivité en acide acrylique reste bonne lorsque le taux de réduction de la composition solide augmente ;
- la composition solide, une fois qu'elle a subi une réduction et une perte progressive de son activité, est facilement régénérable par chauffage en présence d'oxygène ou d'un gaz contenant de l'oxygène après une certaine période d'utilisation ; après la régénération, le solide retrouve son activité initiale et peut être utilisé dans un nouveau cycle de réaction ;
- la séparation des étapes de réduction de la composition solide et de régénération de celle-ci permet :

    - d'augmenter la sélectivité en acide acrylique ; et
    - d'augmenter la pression partielle en acroléine, une telle pression partielle d'alimentation en acroléine n'étant plus limitée par l'existence d'une zone explosive par le mélange acroléine + oxygène.

La présente invention a donc d'abord pour objet l'utilisation d'une composition solide d'oxydes mixtes de formule (I) :

$$Mo_{12}V_aSr_bW_cCu_dSi_eO_x \tag{I}$$

dans laquelle :

- a est compris entre 2 et 14, bornes incluses ;
- b est compris entre 0,1 et 6, bornes incluses ;
- c est compris entre 0 et 12, bornes incluses ;
- d est compris entre 0 et 6, bornes incluses ;
- e est compris entre 0 et 15, bornes incluses ; et
- x est la quantité d'oxygène lié aux autres éléments et dépend de leurs états d'oxydation,

dans la fabrication de l'acide acrylique par oxydation de l'acroléine, ladite composition solide réagissant avec l'acroléine selon la réaction redox (1) :

$$SOLIDE_{oxydé} + ACROLÉINE \rightarrow SOLIDE_{réduit} + ACIDE\ ACRYLIQUE \qquad (1)$$

Les oxydes des différents métaux entrant dans la composition de l'oxyde mixte de formule (I) peuvent être utilisés comme matières premières dans la préparation de cette composition, mais les matières premières ne sont pas limitées aux oxydes ; comme autres matières premières, on peut citer :

- dans le cas du molybdène, le molybdate d'ammonium et l'acide molybdique ;
- dans le cas du vanadium, le métavanadate d'ammonium ;
- dans le cas du strontium, l'hydroxyde, le carbonate ou le nitrate de strontium ;
- dans le cas du tungstène, le tungstate d'ammonium et l'acide tungstique ;
- dans le cas du cuivre, l'hydroxyde, le carbonate ou le nitrate de cuivre ;

et, d'une manière générale, tous les composés susceptibles de former un oxyde par calcination, à savoir sels métalliques d'acides organiques, sels métalliques d'acides minéraux, composés métalliques complexes et composés métalliques organiques, etc.

La source de silicium est généralement constituée par de la silice colloïdale.

Conformément à des modes de réalisation particuliers, on peut préparer des compositions solides de formule (I) en mélangeant sous agitation des solutions aqueuses de paratungstate d'ammonium, de métavanadate d'ammonium, de molybdate d'ammonium, et de nitrate de cuivre et de nitrate de strontium, en ajoutant le cas échéant de la silice colloïdale, puis en les séchant et en les calcinant sous air entre 300 et 600°C, de préférence entre 350 et 500°C.

La présente invention a également pour objet un procédé de fabrication de l'acide acrylique à partir de l'acroléine, procédé suivant lequel on fait passer un mélange gazeux d'acroléine et de vapeur d'eau et, le cas échéant, d'un gaz inerte tel que l'azote, sur une composition solide de formule (I) définie ci-dessus, pour conduire la réaction redox (1) telle qu'indiquée ci-dessus, en opérant à une température de 200 à 500°C, notamment de 250 à 450°C, sous une pression de $1,01 \times 10^4$ à $1,01 \times 10^6$ Pa (0,1 à 10 atmosphères), notamment de $5,05 \times 10^4$ à $5,05 \times 10^5$ Pa (0,5 - 5 atmosphères) et avec un temps de séjour de 0,01 seconde à 90 secondes, notamment de 0,1 seconde à 30 secondes, en l'absence d'oxygène moléculaire.

Le rapport en volume acroléine/vapeur d'eau dans la phase gazeuse n'est pas critique et peut varier dans de larges limites.

Au cours de la réaction redox (1), la composition solide subit une réduction et une perte progressive de son activité. C'est pourquoi, une fois que la composition solide est passée à l'état réduit, on conduit la régénération de ladite composition solide selon la réaction (2) :

$$SOLIDE_{réduit} + O_2 \rightarrow SOLIDE_{oxydé} \qquad (2)$$

par chauffage en présence d'un excès d'oxygène ou d'un gaz contenant de l'oxygène à une température de 250 à 500°C, pendant le temps nécessaire à la réoxydation de la composition solide.

Après la régénération, qui peut être effectuée dans des conditions de température et de pression identiques à ou différentes de celles de la réaction redox, la composition solide retrouve une activité initiale et peut être utilisée dans un nouveau cycle de réaction.

On peut conduire la réaction redox (1) et la régénération dans un dispositif à deux étages, à savoir un réacteur et un régénérateur qui fonctionnent simultanément et dans lesquels alternent périodiquement deux charges de composition solide ; on peut également conduire la réaction redox (1) et la régénération dans un même réacteur en alternant les périodes de réaction et de régénération.

La préparation de l'acide acrylique selon l'invention s'effectue selon une réaction stoechiométrique et non catalytique.

Les Exemples suivants illustrent la présente invention sans toutefois en limiter la portée. Les conversions, sélectivités et rendements sont définis comme suit :

$$Conversion\ (\%) = \frac{Nombre\ de\ moles\ d'acroléine\ ayant\ réagi}{Nombre\ de\ moles\ d'acroléine\ introduites} \times 100$$

$$\text{Sélectivité (\%) en acide acrylique} = \frac{\text{Nombre de moles d'acide acrylique formées}}{\text{Nombre de moles d'acroléine ayant réagi}} \times 100$$

$$\text{Sélectivité (\%) en acide acétique} = \frac{\text{Nombre de moles d'acide acétique formées}}{\text{Nombre de moles d'acroléine ayant réagi}} \times 100$$

EXEMPLE 1(a) :

Préparation d'un solide de formule

$Mo_{12}V_{4,8}Sr_{0,5}W_{2,4}Cu_{2,2}O_x$, x étant la quantité d'oxygène lié aux autres éléments et dépendant de leurs états d'oxydation.

Dans 100 g d'eau, on introduit 3,6 g de paratungstate d'ammonium, 3,0 g de métavanadate d'ammonium et 12,4 g d'heptamolybdate d'ammonium, et on chauffe à 100°C. Dans 5 g d'eau, on introduit 3,0 g de nitrate de cuivre et 0,62 g de nitrate de strontium et on chauffe à 100°C. On ajoute la seconde solution à la première, et la solution résultante est ensuite évaporée à siccité, puis calcinée 4 heures à 400°C.

EXEMPLE 1(b) (comparatif) :

Préparation d'acide acrylique à partir de l'acroléine

On charge 50 mg d'un solide préparé selon l'Exemple 1(a) dans un réacteur tubulaire, puis on le balaye par un flux continu de 20 ml/minute d'air et on le porte à 300°C. Une injection d'une solution aqueuse à 12% en poids d'acroléine, contenant $1,1 \times 10^{-6}$ mole d'acroléine, est envoyée sur le solide. L'acroléine est convertie à 97% avec des sélectivités en acide acrylique et en acide acétique de respectivement 56% et 4%.

EXEMPLE 2 :

Préparation d'acide acrylique à partir de l'acroléine par réaction redox.

Après le traitement de l'Exemple 1(b), on balaye le réacteur par un flux continu de 17 ml/minute d'azote et on le porte à 300°C. Une injection d'une solution aqueuse à 12% en poids d'acroléine, contenant $1,1 \times 10^{-6}$ mole d'acroléine, est envoyée sur le solide. L'acroléine est convertie à 99,1% avec des sélectivités en acide acrylique et en acide acétique de respectivement 65% et 6,5%.

EXEMPLE 3 :

Après avoir conduit la réaction de l'Exemple 2, on soumet à nouveau le même solide à onze injections successives d'acroléine dans les mêmes conditions de test qu'à l'Exemple 2. Les performances obtenues sont rapportées dans le Tableau 1. (L'injection n' 1 correspond à l'Exemple 2).

Tableau 1

| N° d'injection | Conversion de l'acroléine (%) | Sélectivité en acide acrylique (%) | Sélectivité en acide acétique (%) |
|---|---|---|---|
| 1 | 99,1 | 65 | 6,5 |
| 2 | 98,2 | 79 | 6,5 |
| 3 | 96,5 | 81 | 5,9 |
| 4 | 95,7 | 86 | 5,3 |
| 5 | 92,8 | 89 | 3,9 |
| 6 | 89,8 | 90 | 3,6 |
| 7 | 85,5 | 90 | 3,3 |
| 8 | 83,4 | 92 | 3,1 |
| 9 | 83,9 | 91 | 3,4 |

Tableau 1   (suite)

| N° d'injection | Conversion de l'acroléine (%) | Sélectivité en acide acrylique (%) | Sélectivité en acide acétique (%) |
|---|---|---|---|
| 10 | 84,0 | 89 | 3,4 |
| 11 | 73,6 | 90 | 3,6 |
| 12 | 70,8 | 91 | 3,6 |

EXEMPLE 4 :

Après le traitement réducteur de l'Exemple 3, le solide est régénéré 2 heures à 300°C sous flux d'air, puis replacé sous flux d'azote. Vingt-quatre nouvelles injections successives de solution aqueuse à 12% en poids d'acroléine, contenant $1,1 \times 10^{-6}$ mole d'acroléine, sont envoyées sur le solide.

Les performances obtenues sont rapportées dans le Tableau 2.

Tableau 2

| N° d'injection | Conversion de l'acroléine (%) | Sélectivité en acide acrylique (%) | Sélectivité en acide acétique (%) |
|---|---|---|---|
| 1 | 99,5 | 61 | 5,3 |
| 2 | 90,8 | 79 | 3,8 |
| 3 | 97,7 | 84 | 5,3 |
| 4 | 94,2 | 90 | 4,0 |
| 5 | 93,0 | 88 | 3,8 |
| 6 | 81,4 | 89 | 3,5 |
| 7 | 83,2 | 91 | 2,9 |
| 8 | 82,3 | 90 | 3,0 |
| 9 | 82,4 | 90 | 3,3 |
| 10 | 78,6 | 90 | 3,4 |
| 11 | 71,4 | 91 | 3,6 |
| 12 | 68,5 | 89 | 3,8 |
| 13 | 67,8 | 90 | 3,7 |
| 14 | 62,5 | 90 | 3,7 |
| 15 | 61,5 | 89 | 3,7 |
| 16 | 58,1 | 90 | 3,8 |
| 17 | 56,2 | 92 | 3,8 |
| 18 | 54,7 | 91 | 3,7 |
| 19 | 54,1 | 91 | 3,7 |
| 20 | 49,1 | 92 | 3,9 |
| 21 | 47,5 | 92 | 3,9 |
| 22 | 49,1 | 92 | 3,7 |
| 23 | 47,8 | 93 | 3,7 |
| 24 | 42,6 | 94 | 3,9 |

**Revendications**

1.  Utilisation d'une composition solide d'oxydes mixtes de formule (I) :

$$Mo_{12}V_aSr_bW_cCu_dSi_eO_x \qquad (I)$$

dans laquelle :

-   a est compris entre 2 et 14, bornes incluses ;
-   b est compris entre 0,1 et 6, bornes incluses ;
-   c est compris entre 0 et 12, bornes incluses ;
-   d est compris entre 0 et 6, bornes incluses ;
-   e est compris entre 0 et 15, bornes incluses ; et
-   x est la quantité d'oxygène lié aux autres éléments et dépend de leurs états d'oxydation,

dans la fabrication de l'acide acrylique par oxydation de l'acroléine, ladite composition solide réagissant avec l'acroléine selon la réaction redox (1) :

$$SOLIDE_{oxydé} + ACROLÉINE \rightarrow SOLIDE_{réduit} + ACIDE\ ACRYLIQUE \qquad (1)$$

2.  Procédé de fabrication de l'acide acrylique à partir de l'acroléine, caractérisé par le fait que l'on fait passer un mélange gazeux d'acroléine et de vapeur d'eau et, le cas échéant, d'un gaz inerte sur une composition solide de formule (I) telle que définie à la revendication 1, pour conduire la réaction redox (1) telle qu'indiquée à la revendication 1, en opérant à une température de 200 à 500°C, sous une pression de $1,01 \times 10^4$ à $1,01 \times 10^6$ Pa (0,1 à 10 atmosphères), et avec un temps de séjour de 0,01 seconde à 90 secondes, en l'absence d'oxygène moléculaire.

3.  Procédé selon la revendication 2, caractérisé par le fait que l'on conduit la réaction redox (1) à une température de 250 à 450°C.

4.  Procédé selon l'une des revendications 2 et 3, caractérisé par le fait que l'on conduit la réaction redox (1) sous une pression de $5,05 \times 10^4$ - $5,05 \times 10^5$ Pa (0,5 - 5 atmosphères).

5.  Procédé selon l'une des revendications 2 à 4, caractérisé par le fait que l'on conduit la réaction redox (1) avec un temps de séjour de 0,1 seconde à 30 secondes.

6.  Procédé selon l'une des revendications 2 à 5, caractérisé par le fait qu'une fois que la composition solide est passée à l'état réduit, on conduit la régénération de ladite composition solide selon la réaction (2) :

$$SOLIDE_{réduit} + O_2 \rightarrow SOLIDE_{oxydé} \qquad 2)$$

par chauffage en présence d'un excès d'oxygène ou d'un gaz contenant de l'oxygène à une température de 250 à 500°C, pendant le temps nécessaire à la réoxydation de la composition solide.

7.  Procédé selon la revendication 6, caractérisé par le fait que l'on conduit la réaction redox (1) et la régénération dans un dispositif à deux étages, à savoir un réacteur et un régénérateur qui fonctionnent simultanément et dans lesquels alternent périodiquement deux charges de composition solide.

8.  Procédé selon la revendication 6, caractérisé par le fait que l'on conduit la réaction redox (1) et la régénération dans un même réacteur en alternant les périodes de réaction et de régénération.

**Patentansprüche**

1. Verwendung einer festen Zusammensetzung gemischter Oxide der Formel (I)

$$Mo_{12}V_aSr_bW_cCu_dSi_eO_x \tag{I},$$

in der

- a im Bereich von 2 bis 14 einschließlich der Grenzwerte,
- b im Bereich von 0,1 bis 6 einschließlich der Grenzwerte,
- c im Bereich von 0 bis 12 einschließlich der Grenzwerte,
- d im Bereich von 0 bis 6 einschließlich der Grenzwerte,
- e im Bereich von 0 bis 15 einschließlich der Grenzwerte liegt und
- x die Menge an Sauerstoff ist, die mit den anderen Elementen

verbunden ist und von ihrem Oxidationszustand abhängt, zur Herstellung von Acrylsäure durch Oxidation von Acrolein, wobei die feste Zusammensetzung mit dem Acrolein gemäß der Redox-Reaktion (1)

$$\text{Feststoff}_{oxidiert} + \text{Acrolein} \rightarrow \text{Feststoff}_{reduziert} + \text{Acrylsäure} \tag{1}$$

reagiert.

2. Verfahren zur Herstellung von Acrylsäure aus Acrolein, dadurch gekennzeichnet, daß man ein gasförmiges Gemisch aus Acrolein und Wasserdampf und gegebenenfalls einem inerten Gas über/durch eine wie in Anspruch 1 definierte feste Zusammensetzung der Formel (I) strömen läßt, um die wie in Anspruch 1 angegebene Redox-Reaktion (1) durchzuführen, wobei die Reaktion bei einer Temperatur von 200 bis 500 °C und einem Druck von $1,01 \cdot 10^4$ bis $1,01 \cdot 10^6$ Pa (0,1 bis 10 atm) und mit einer Verweilzeit von 0,01 bis 90 s in Abwesenheit von molekularem Sauerstoff durchgeführt wird.

3. Verfahren nach Anspruch 2, dadurch gekennzeichnet, daß die Redox-Reaktion (1) bei einer Temperatur von 250 bis 450 °C durchgeführt wird.

4. Verfahren nach einem der Ansprüche 2 und 3, dadurch gekennzeichnet, daß die Redox-Reaktion (1) bei einem Druck von $5,05 \cdot 10^4$ bis $5,05 \cdot 10^5$ Pa (0,5 bis 5 atm) durchgeführt wird.

5. Verfahren nach einem der Ansprüche 2 bis 4, dadurch gekennzeichnet, daß die Redox-Reaktion (1) mit einer Verweilzeit von 0,1 bis 30 s durchgeführt wird.

6. Verfahren nach einem der Ansprüche 2 bis 5, dadurch gekennzeichnet, daß nach Überführung der festen Zusammensetzung in den reduzierten Zustand die Regeneration der festen Zusammensetzung gemäß der Reaktion (2)

$$\text{Feststoff}_{reduziert} + O_2 \rightarrow \text{Feststoff}_{oxidiert} \tag{2}$$

durch Erhitzen in Gegenwart eines Überschusses an Sauerstoff oder eines Gases, das Sauerstoff enthält, auf eine Temperatur von 250 bis 500 °C während eines Zeitraums, der für die Oxidation der festen Zusammensetzung erforderlich ist, durchgeführt wird.

7. Verfahren nach Anspruch 6, dadurch gekennzeichnet, daß die Redox-Reaktion (1) und die Regeneration in einer Vorrichtung mit zwei Bereichen, nämlich einem Reaktor und einem Regenerator, durchgeführt werden, die gleichzeitig in Betrieb sind und zwischen denen zwei Chargen der festen Zusammensetzung periodisch wechseln.

8. Verfahren nach Anspruch 6, dadurch gekennzeichnet, daß die Redox-Reaktion (1) und die Regeneration in einem einzigen Reaktor durchgeführt werden, wobei die Zeitintervalle, in denen die Reaktion bzw. die Regeneration stattfindet, einander abwechseln.

**EP 0 861 821 B1**

**Claims**

1. Use of a solid mixed oxides composition of formula (I):

$$Mo_{12}V_aSr_bW_cCu_dSi_eO_x \tag{I}$$

in which:

- a is between 2 and 14, limits included,
- b is between 0.1 and 6, limits included,
- c is between 0 and 12, limits included,
- d is between 0 and 6, limits included,
- e is between 0 and 15, limits included, and
- x is the quantity of oxygen bonded to the other elements and depends on their oxidation state,

in the manufacture of acrylic acid by oxidation of acrolein, the said solid composition reacting with the acrolein according to the redox reaction (1):

$$SOLID_{oxidized} + ACROLEIN \rightarrow SOLID_{reduced} + ACRYLIC\ ACID \tag{1}.$$

2. Process for the manufacture of acrylic acid from acrolein, characterized in that a gaseous mixture of acrolein and of water vapour and, if appropriate, of an inert gas is passed over a solid composition of formula (I) as defined in Claim 1, to conduct the redox reaction (1) as indicated in Claim 1, by operating at a temperature of 200 to 500°C, at a pressure of $1.01 \times 10^4$ to $1.01 \times 10^6$ Pa (0.1 to 10 atmospheres) and with a residence time of 0.01 second to 90 seconds, in the absence of molecular oxygen.

3. Process according to Claim 2, characterized in that the redox reaction (1) is conducted at a temperature of 250 to 450°C.

4. Process according to either of Claims 2 and 3, characterized in that the redox reaction (1) is conducted at a pressure of $5.05 \times 10^4$ - $5.05 \times 10^5$ Pa (0.5 - 5 atmospheres).

5. Process according to one of Claims 2 to 4, characterized in that the redox reaction (1) is conducted with a residence time of 0.1 second to 30 seconds.

6. Process according to one of Claims 2 to 5, characterized in that, once the solid composition has changed to the reduced state, regeneration of the said solid composition is conducted according to the reaction (2):

$$SOLID_{reduced} + O_2 \rightarrow SOLID_{oxidized} \tag{2}$$

by heating in the presence of an excess of oxygen or of an oxygen-containing gas at a temperature of 250 to 500°C, for the time needed for the reoxidation of the solid composition.

7. Process according to Claim 6, characterized in that the redox reaction (1) and the regeneration are conducted in a two-stage device, namely a reactor and a regenerator which operate simultaneously and in which two charges of solid composition alternate periodically.

8. Process according to Claim 6, characterized in that the redox reaction (1) and the regeneration are conducted in the same reactor by alternating the reaction and regeneration periods.